Europäisches Patentamt

European Patent Office

Office européen des brevets

(11) Publication number: **0 172 747**
**A2**

## (12) EUROPEAN PATENT APPLICATION

(21) Application number: 85305904.6

(22) Date of filing: 20.08.85

(51) Int. Cl.⁴: **A 61 B 5/02**
**A 61 B 5/22**

(30) Priority: 24.08.84 JP 175179/84

(43) Date of publication of application:
26.02.86 Bulletin 86/9

(84) Designated Contracting States:
DE FR GB

(71) Applicant: Citizen Watch Co. Ltd.
2-1-1 Nishi-Shinjuku
Shinjuku-Ku Tokyo(JP)

(71) Applicant: NIPPON KOKAN KABUSHIKI KAISHA
1-2 Marunouchi 1-chome Chiyoda-ku
Tokyo 100(JP)

(72) Inventor: Murakami, Tomomi
2-3-58 Tamako-cho Higashimurayama-shi
Tokyo(JP)

(72) Inventor: Makoshi, Michiyuki
1-9-24 Jiyugaoka
Meguro-ku Tokyo(JP)

(74) Representative: Eurolink
Regent House Heaton Lane
Stockport Cheshire, SK4 1BS(GB)

(54) Pulse rate monitor.

(57) Disclosed is a pulse rate monitor having means for electro-optically detecting any variation of blood flow rate in the capillaries of earlobe; means for determining in terms of pulse rates so to which rank an exercise taken by a user belongs to; and means for informing him of the result of the decision thus made with the aid of sound or artificial voice using words.

FIG. 4

Croydon Printing Company Ltd.

### TITLE OF THE INVENTION

Pulse rate monitor

### BACKGROUND OF THE INVENTION

FIELD OF THE INVENTION

The present invention relates to a pulse rate monitor for monitoring user's pulse rates during a exercise.

During recent years, an increasing number of people have been engaging in regular exercise in order to keep fit. It is said that exercise appropriate for the purpose of keeping fit is hard enough to keep pulse rates ranging from 70 to 80 percent of maximum pulse rates. In an attempt to meet a desire for measuring pulse rates during exercise wrist watches equipped with pulse rate monitors have been produced and been commercially available.

There are two different kinds of pulse rate monitors that is, one using a photoelectric type detector and the other using an electrocardiograph type detector. In the former type detector pulse rates are determined electro-optically in terms of blood flow rates in finger with a finger put on a photo-electric sensor, which is fixed to the casing of a wrist-watch. In the latter type detector pulse rates are determined in terms of electric potential appearing on finger with a finger put on a cardiograph sensor, which is fixed to the casing of a wrist-watch, too. Otherwise, pulse rates are determined in terms of electric potential appearing on breast. An electrode is fitted on breast, and a length of thin electric cord extends from the electrode on a user's breast to the pulse rate monitor-built in wrist-watch. In either type the pulse rate is digitally displayed in the face of the wrist-watch.

As for the type in which a sensor or electrode is provided to the casing of a wrist watch a user who is taking an exercise, for instance, jogging, must put his finger on the sensor or electrode all the time during jogging. This posture is inconvenient to jogging. Also, it is difficult for him to put his finger on the sensor or electrode in a stable way in the course of jogging, because his body is moving all the time, and therefore no stable measurement is possible.

As for the type in which a thin electric cord is used to connect an electrode on breast to a wrist watch, a stable measurement is assured, but the electric cord constitutes a hindrance to jogging or any other exercise.

As regards the way of informing users of their pulse rates during exercise, they must bend and raise their arms to have a look at the digital display of pulse rates in the faces of their wrist watches. This posture is inconvenient to the continuance of jogging or any other exercise. Also, the act of watching the digital display of pulse rates in the face of a wrist watch is difficult because a user's body is moving to cause a constant tremble on the wrist watch.

For these reasons wrist watches equipped with pulse rate monitors now commercially available are not satisfactory, and there is a strong desire for appearance of pulse rate monitors capable of measuring pulse rates during exercise in a stable way and at the same time capable of informing users of their pulse rates without trouble.

## SUMMARY OF THE INVENTION

One object of the present invention is to provide a pulse rate monitor which is capable of using different sounds or artificial voices for informing a user of his pulse rates.

Another object of the present invention is to provide a pulse rate monitor which is adapted to fit around ear.

Still another object of the present invention is to provide a pulse rate monitor which is capable of informing a user of the load of exercise he is taking by means of different sounds of artificial voice telling him that you are taking a sufficient exercise; you are taking an insufficient exercise; or you are taking an excessive exercise.

Still another object of the present invention is to provide a pulse rate monitor which is capable of informing a user as to which rank the exercise he is taking belongs to by means of sounds or artificial voice, the ranks being set forth different age groups.

BRIEF DESCRIPTION OF THE DRAWINGS

Fig. 1 is a graph representing the relationship between pulse rates and different age groups for different levels of exercise load;

Fig. 2 shows a pulse rate monitor of ear hanging type in which the present invention is preferably embodied;

Fig. 3 is a longitudinal section of the pulse monitor taken along the line A-A in Fig. 2;

Fig. 4 is a block diagram of an example of an electric circuit used in a pulse rate monitor according to the present invention;

Fig. 5 shows waveforms of signals appearing at different portions in the circuit of Fig. 4; and

Fig. 6 is a block diagram of another example of an electric circuit used in a pulse rate monitor according to the present invention.

DETAILED DESCRIPTION OF PREFERRED EMBODIMENTS

It is said that when one is taking an effective exercise, his pulse rates range from 60 to 80 percent of the maximum pulse rate, which is dependent upon physical strength of individuals, but may be justly estimated by the following equation:

Maximum pulse rate = 210 - 0.8 x age

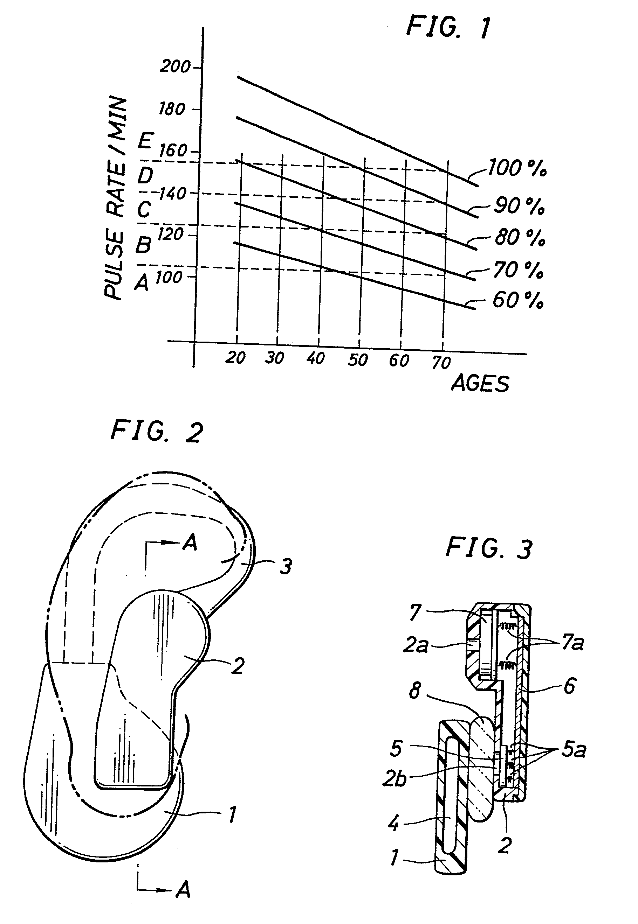

Fig. 1 is a graph showing the relationship between pulse rates and different age groups for different exercise loads, that is, 60, 70, 80, 90 and 100 percent of maximum pulse rate. Also, pulse rates or numbers of pulses per minute (ordinate) are divided into different groups, that is, Rank "A" (below 105 pulses/minute), Rank "B" (105 to 125 pulses/minute), Rank "C" (125 to 140 pulses/minute), Rank "D" (140 to 155 pulses/minute) and Rank "E" (above 155 pulse/minute) as indicated by broken lines.

Table 1 shows the relationship between these ranks and age groups for sufficient exercise (double circle), somewhat excessive exercise and insufficient exercise (circle) and excessive exercise (cross).

Table 1

| | Age Groups | | | | |
|---|---|---|---|---|---|
| Ranks | 20 to 29 | 30 to 39 | 40 to 49 | 50 to 59 | 60 to 69 |
| E | X | X | X | X | X |
| D | ◎ | ○ | X | X | X |
| C | ◎ | ◎ | ◎ | ○ | X |
| B | ◎ | ◎ | ◎ | ◎ | ◎ |
| A | ○ | ○ | ○ | ◎ | ◎ |

This table represents one example showing sufficient exercise (double circle) for different age groups. During exercise a user can be acoustically or talken as to whether he is taking a sufficient exercise or not according to this table with the aid of sound generating devices such as a buzzer or of an artificial voice generating device.

Fig. 2 shows an ear hanging type pulse rate monitor in which the present invention is preferably embodied.

In Fig. 2 an electric cell casing 1 and a pulse rate detector housing 2 are connected with each other by a joint member 3, which is bent so as to be easy to hang on an ear (broken lines). The electric cell casing 1 contains an electric cell as a power supply for the monitor. The pulse rate detector housing 2 contains a pulse rate sensor, an electronic circuit for calculating pulse signals from the sensor, and for making a decision as to which rank the pulse rate belongs to, and a buzzer or any other sound producing device for producing sound signal or artificial voice to inform the user that he is taking a sufficient exercise (or insufficient or excessive exercise). The joint member 3 mechanically and electrically connects the cell casing 1 and the pulse rate detector housing 2 with each other. The joint member 3 contains a thin electric wire connecting the electric cell to the detector. As shown in the drawing, the pulse rate monitor may be attached around ear with its joint member 3 around the upper part of the ear, its detector housing 2 and cell casing 1 pinching the earlobe. Fig. 3 shows a longitudinal section taken along the line A-A of

Fig. 2. As shown in the drawing, an electric cell 4 is contained in the cell casing 1. A pulse rate sensor 5 is composed of a light-emitting diode and a photo transister for detecting the pulsation of blood flow in the capillaries of the earlobe. The pulse rate sensor 5 is connected to an associated circuit 6 by means of springs 5a.

The circuit plate 6 bears an electric circuit for performing an arithmetic operation on the signals from the pulse rate sensor 5; making a decision as to which rank the pulse rate belongs to; and generating a sound signal. The sound signal is converted to sound by a buzzer 7. The buzzer 7 is connected to the circuit plate 6 by means of connection spring 7a. Sound from the buzzer 7 is released from apertures 2a. The light from the light emitting diode passes through a window-opening 2b, and it reflects from the capillaries in the earlobe 8, to fall on the photo transistor through the window-opening 2b.

As is seen from Fig. 3, the earlobe 8 is sandwiched between the pulse rate detector housing 2 and the electric cell casing 1. With this arrangement the amount of reflection of the near-infrared light falling on the photo-transistor of the pulse rate sensor 5 varies with the blood flow rate in the capillaries of the earlobe 8. The photo-transistor in the pulse rate sensor 5 is responsive to the variation of the reflection of the near-infrared light for detecting pulse rates in terms of blood flow rate. Sound generating signals representing the results of calculation of the pulse rates thus detected are directed to the buzzer 7 via the buzzer connection spring 7a, and then the buzzer 7 generates different sounds for different ranks of pulse rates.

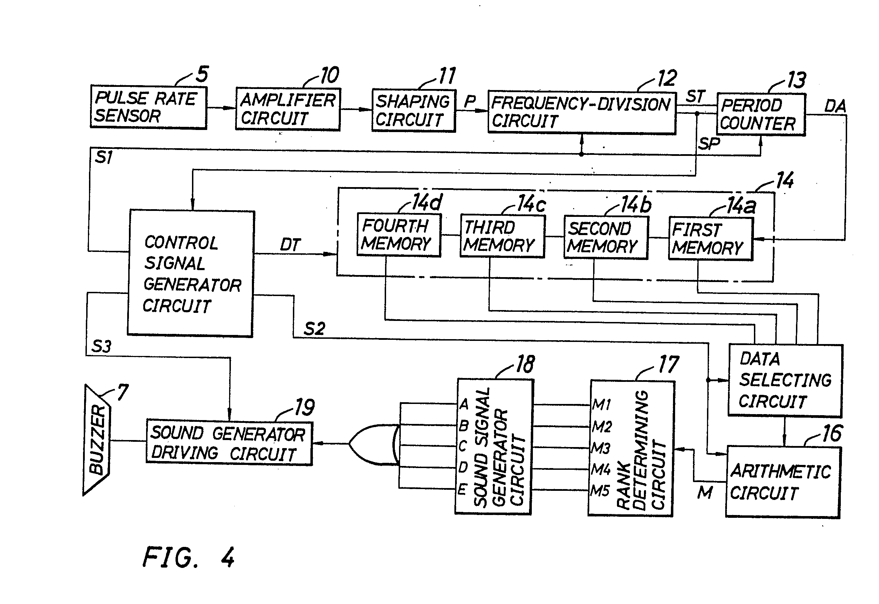

Now, referring to Fig. 4, a circuit arrangement of the pulse rate monitor as shown in Figs. 2 and 3 is shown as comprising a pulse rate sensor 5 using a photo-coupler which is described above with reference to Fig. 2; an amplifier circuit 10 for amplifying a signal of very small amplitude from the pulse rate sensor 5; a shaping circuit 11 for shaping sensor pulse signals from the amplifier 10; a frequency-division circuit 12 for providing a measurement-starting signal ST and a measurement-ending signal SP alternately for each sensor pulse P; a period counter 13 for determining the intervals between adjacent sensor pulse signals P under the control of the measurement-starting and measurement-ending signals ST and SP; a data memory 14 for storing measurement data DA from the period counter 13, said data memory 14 being composed of a first memory 14a, a second memory 14b, a third memory 14c and a fourth memory 14d all responsive to data-transfer signals DT for shifting data from memory to memory in the order named, thus storing latest four data all the time; a data selecting circuit 15 for comparing data stored in these four memories 14a to 14d, eliminating maximum and minimum data and selecting two intermediate data only; an arithmetic circuit 16 for determining the average value for these intermediate data and providing an average value M of pulse rates; a rank determining circuit 17 for determining as to which rank of M1 to M5 the exercise belongs to on the basis of the average value of the pulse rate; a sound signal generator circuit 18 responsive to the decision of rank thus made by the rank determining circuit 17 for providing five different sound generating signals A to E; a sound generator driving circuit 19 responsive to the sound generating signal from the sound signal generator circuit 18 for driving an associated buzzer 7; and a control signal generator circuit 20 for generating timing pulses $S_1$, $S_2$ and $S_3$ for controlling measurement of pulse rates, calculation, sound-generating and any other necessary operations in these circuits.

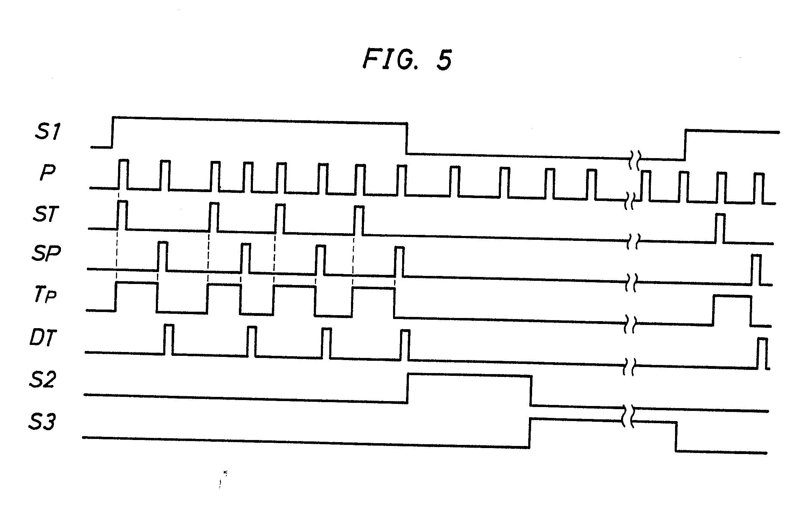

The operation of the pulse rate monitor is described below with reference to Fig. 5.

An electric signal representing the variation of blood flow in earlobe is directed from the pulse rate sensor 5 to the shaping circuit 11 via the amplifier circuit 10, and a train of sensor pulses P appear at the output terminal of the shaping circuit 11. As shown in Fig. 5, assume that these sensor pulse signals P appear at the output terminal of the shaping circuit 11. If the measurement signal $S_1$ supplied by the control signal generator circuit 20 turns to high level "H", the frequency divider 12 and the period counter 13 are brought in operation. Then, the frequency divider 12 provides to the period counter 13 a measurement starting signal ST and a measurement ending signal SP for every other sensor pulse P, as seen from Fig. 5. Accordingly, the period counter 13 determines intervals $T_p$ between adjacent sensor pulses P, providing measurement data DA. A measurement ending signal SP is directed to the control signal generator 20 at the termination of measurement.

After a short delay from application of the measurement ending signal SP to the control signal generator circuit 20, it supplies a data transfer signal DT to the data memory 14. The application of the data transfer signal DT to the data memory 14 causes sequential shift of data from the first memory 14a to the second memory 14b; from the second memory 14b to the third memory 14c; and from the third memory 14c to the fourth memory 14d, and then the latest data DA provided by the period counter 13 occupies the first memory 14a. The application of another measurement starting signal ST to the period counter 13 causes resetting of the period counter 13 for measurement. Thus, after the data memory 14 has stored latest four data DA, the control signal generator circuit 20 sends an arithmetic operation instructing signal S2 both to the data selecting circuit 15 and to the arithmetic operation circuit 16, thus bringing them in operation. Then, the data selecting circuit 15 compares four data with each other to select and supply two intermediate data to the arithmetic operation circuit 16. In the arithmetic operation circuit 16 an average value M of pulse rates is calculated from the so supplied two data, and the result of calculation is directed to the rank determining circuit 17. Then the arithmetic operation instructing signal S2 turns to low level "L" whereas a heartrate warning signal S3 turns to high level "H". The rank determining circuit 17 determines as to which rank the average value of pulse rates belongs to, thus selecting one among signals M1 to M5 and supplying the signal thus selected to the sound signal generator circuit 18. Accordingly, the sound signal generator circuit 18 selects an appropriate one among different sound generating signals A to E, and supplies the so selected signal to the sound generator driving circuit 19. Then, the buzzer generates a beeping signal while the warning signal S3 lasts. As for the sound generating signals A to E, they may be different in frequency, or may be artificial voices telling the user the degree of exercise.

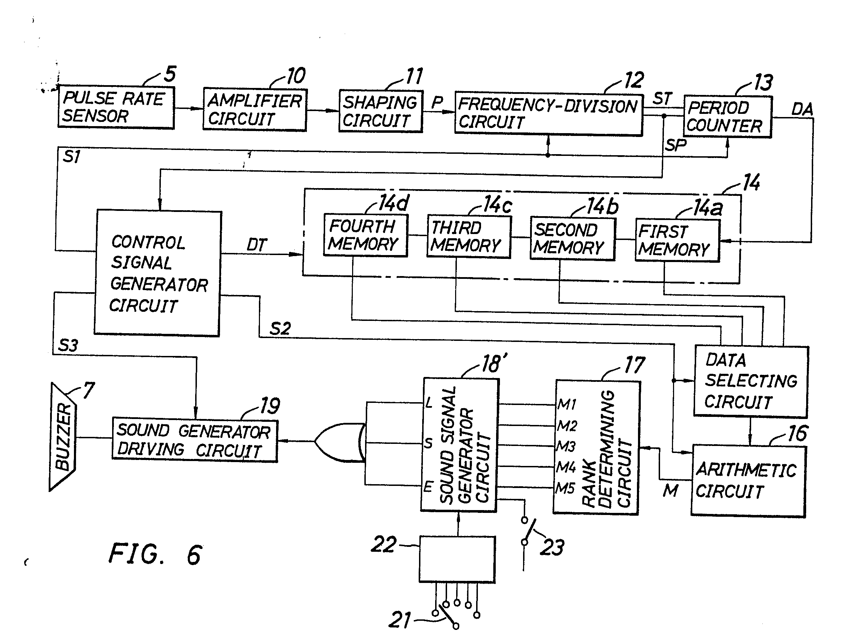

Fig. 6 shows a pulse rate monitor circuit according to another embodiment of the present invention. The pulse rate monitor circuit is designed so as to inform a user as to whether he is taking an insufficient exercise, a sufficient exercise or an excessive exercise for his age group with the aid of sound or artificial voice particularly alerting him to slow down when he is taking an excessive exercise. Thus, the pulse rate monitor of Fig. 5 has an age setting switch 21, an age setting circuit 22 for generating age signals, and a warning switch 23 as extra units which do not appear in Fig. 4.

The age setting switch 21 is of a rotary type, and is designed for a user to set for an age group which his age belongs to (20s to 60s). Specifically by selecting and pushing an appropriate button the age setting circuit 22 provides an age signal representing the age group thus selected to the sound signal generator 18'. The sound signal generator 18' provides a sound generating signal representing an insufficient exercise zone at an output terminal marked "L", an appropriate exercise zone at an output terminal marked "S" or an excessive exercise zone at an output terminal marked "E" of the sound signal generator 18' according to Table 2.

Table 2

| Ranks | Ages | | | | |
|---|---|---|---|---|---|
| | 20s | 30s | 40s | 50s | 60s |
| E | E | | | | |
| D | | E | E | E | E |
| C | S | | | | |
| B | | S | S | | |
| A | L | L | L | S | S |

The drive circuit 19 provides to the buzzer 7 signals of different frequencies each allotted to each of insufficient appropriate and excessive exercises. Then, the buzzer 7 generates a beeping signal to inform the user of the degree or load of exercise estimated in terms of his pulse rate, taking his age into consideration.

If the alarming switch 23 is made to turn on, signals are prevented from appearing at the output terminals L and S of the sound signal generator circuit 18', allowing a signal to appear only at the output terminal E of the sound signal generator 18'. Then, the beeping signal from the buzzer is used as alarming only, and therefore the user when alarmed slows down, or takes a rest. When using an artificial voice, it will say, for example, "Slow down, please".

A pulse rate monitor according to the present invention is described above as putting around ear, but it should be understood that it can be made in the form of finger ring

0172747

or fingerstall.

As is apparent from the above, in a pulse rate monitor according to the present invention, different sound signals each representing different exercise loads are sent directly to ear, thereby permitting the user to control his exercise all the time. An ear hanging type pulse rate monitor according to the embodiment of the present invention requires no inconvenient operation for detecting pulse rates during exercise.

WHAT IS CLAIMED IS:

1. A pulse rate monitor comprising:

a sensor for detecting any variation of blood flow rate in a human body;

a rank determining circuit for determining to which rank of the predetermined number of ranks the pulse rate detected in terms of blood flow rate belongs to;

a sound signal generator responsive to rank signals from said rank determining circuit for generating sound generating signals representing the ranks represented by the rank signals; and

a sound generating unit responsive to the sound generating signals from said sound signal generator for generating different sounds representing the pulse rates, thereby informing a user of his pulse rate by means of different sound.

2. A pulse rate monitor according to claim 1, wherein said sensor is composed of an optical sensor for detecting any variation of blood flow rate in a human body.

3. A pulse rate monitor according to claim 1 or 2, wherein said monitor is adapted to fit around ear, and said sensor is adapted to push itself against a part of ear for detecting any variation of blood flow rate therein.

4. A pulse rate monitor according to claim 1, wherein it further comprises age setting means, said sound generating unit being driven to generate sounds at different frequencies depending on the age groups selected by said age setting means.

5. A pulse rate monitor according to claim 1, wherein it further comprises means for inhibiting at least one sound generating signal.

6. A pulse rate monitor according to claim 4, wherein said sound signal generator is controlled by said age setting means so as to provide pieces of information of different loads of exercise estimated for different age groups, such as insufficient exercise, appropriate exercise or excessive exercise with the aid of different sounds or artificial voice.

*FIG. 1*

*FIG. 2*

*FIG. 3*

FIG. 4

# FIG. 5

FIG. 6